# EUROPEAN PATENT SPECIFICATION

(11) **EP 0 382 355 B1**
(45) Date of publication and mention of the grant of the patent: **20.08.1997**
(21) Application number: 90300548.6
(22) Date of filing: 18.01.1990
(51) Int. Cl.: A61K 31/715, A61K 31/70, A23K 1/00, C12N 1/20

(54) **Growth promoting agent for bacteria containing pullulan with or without dextran**
Bakteriumwachstumsfördernde Substanz enthaltend Pullulan mit oder ohne Dextran
Agent favorisant la croissance des bactéries contenant du pullulan avec ou sans dextrane

(30) Priority: 09.02.1989 JP 28661/89; 14.12.1989 JP 322564/89
(43) Date of publication of application: 16.08.1990
(73) Proprietor: KABUSHIKI KAISHA HAYASHIBARA SEIBUTSU KAGAKU KENKYUJO, Okayama-shi Okayama (JP)
(72) Inventor: Mitsuhashi, Masakazu, Okayama-shi, Okayama (JP); Yoneyama, Masaru, Souja-shi, Okayama (JP); Sakai, Shuzo, Seto-cho Akaiwa-gun, Okayama (JP)
(74) Representative: Pendlebury, Anthony

(56) References cited:
- EP-A- 0 153 013
- EP-A- 0 363 481
- WO-A-88/00830
- CHEM. PHARM. BULL., vol. 26, no. 1, 1978, pages 3306-3311; K. YAZAWA et al.: "Oligosaccharides and polysaccharides specifically utilizable by bifidobacteria"
- INFECT. IMMUN., vol. 42, no. 2, 1983, pages 716-720, American Society for Microbiology; A.G. KASTER et al.: "Extracellular dextranase activity produced by human oral strains of the genus Bifidobacterium"

## Description

The present invention relates to a growth-promoting agent for bifid bacteria, more particularly, to that containing pullulan and/or dextran as an effective component.

Bifid bacteria are gram positive polymorphic obligate anaerobes and grouped into the genus Bifidobacterium in Bergey's Manual of Systematic Bacteriology, first edition, pp.1418-1434 (1984), published by Williams & Wilkins Co., U.S.A. Bifid bacteria are nonpathogenic and usually live in the large intestines of human and animals, forming intestinal bacterial flora with other microorganisms. Infants' intestinal bacterial flora is mainly of bifid bacteria. After the weaning period that complicates the preference of meals, the ratio of bifid bacteria lowers little by little. In the case of adult, the ratio usually lowers to about 10%, occasionally, to a level wherein no bifid bacterium is detected.

It has been known that bifid bacteria behave in intestines as described below and play an extremely important role in the maintenance and improvement of health and beauty:
(1) Bifid bacteria produce organic acids such as acetic acid and lactic acid, thus decreasing intestinal pH level and suppressing various infectious bacteria;
(2) Bifid bacteria suppress the growth of putrefactive bacteria, thus suppressing the production of harmful substances such as ammonia, indol, cresol and paracresol which tend to occur during the metabolism of amino acids and proteinous substances;
(3) Bifid bacteria appropriately stimulate intestines and promote their peristalsis, thus restoring intestinal order;
(4) Bifid bacteria biologically synthesize vitamins; and
(5) Bifid bacteria prevent and/or treat hyperammonemia and hepatic encephalopathy.

The applicants initiated the investigation of methods which increase the ratio of bifid bacteria in intestinal bacterial floras. For example, some methods attempt to administer intact bifid bacteria. Although these methods may temporally increase the ratio of bifid bacteria, they are unsuccessful in providing a consistent habitat for bifid bacteria and maintaining an increased ratio in intestinal bacterial flora.

Recently proposed is a method wherein various saccharides are orally administered as growth-promoter for intestinal bifid bacteria.

Some methods propose the administration of oligosaccharides such as lactulose, raffinose, lactosucrose (or O-β-D-galactosyl-(1→4)-O-α-D-glucosyl-(1←2)-β-D-fructoside), fructooligosaccharide, galactooligosaccharide and xylooligosaccharide. These oligosaccharides, however, have disadvantages that they have an undesired sweetness, and that their effective dose greatly varies dependently on individuals and this renders their administration range narrow: A small dose leads to no effect, while an excessive intake may cause diarrhea. Furthermore, the oligosaccharides are generally highly hygroscopic, unstable against heat and inferior in processibility.

Other methods propose the use of tasteless high molecular polysaccharides. For example, Japanese Patent Laid-Open No.165,325/88 proposes the use of hemicelluloses, more particularly, heteroglycan or a complex of cereal arabinose and xylose. The practice of this proposal is, however, very difficult because the preparation of such heteroglycan is complicated and its yield is unsatisfactorily low.

Chemical Pharmaceutical Bulletin, Vol.26, No.11, pp.3306-3311 (1978) reports in detail the utilization of simple polysaccharides or homoglycans. According to the article, comparative experiments on various simple polysaccharides for their growth-promoting effect on bifid bacteria revealed that inulin (a type of homofructan) exerted a significant growth-promoting effect, but homoglucans such as pullulan, dextran and soluble starch exerted no such effect.

W88/008830 discloses a method of treatment of a patient suffering from hepatic renal failure, which comprises administration of an effective amount of dextran of a weight average molecular weight in the range 10,000 to 50 million to a patient suffering from such a condition.

EP-A-0153013 discloses a formulation suitable for enteral administration which comprises a bulking proportion of a dextran having an average molecular weight of from 10,000 to 50 million for controlling the calorie intake of a patient, especially one suffering from diabetes.

EP-A-0363481 which is to be considered pursuant to Art. 54(3) and (4) EPC discloses a food containing edible fibres for facilitating bowel movement, which edible fibres include hemicellulose together with water-soluble dietary fibres such as polydextrose, pullulan, gum arabic, tamarind gum, pectin, guar gum, glucomannan and the like.

Although as described above homoglucans were reported to show no growth-promoting effect on bifid bacteria, the present applicants found non-toxic and harmless homoglucans with a high degree of safety and screened out those which exhibited a significant growth-promoting effect on bifid bacteria.

The present applicants studied in detail the above article and found that the experiments revealing the absence of growth-promoting effect were all carried out in vitro and taught nothing about their behaviour in vivo. Thus, the present applicants conducted field-tests on highly safe pullulan, dextran and soluble starch directly using an in vivo system. The field tests resulted in an unexpected finding that pullulan, dextran and soluble starch had a significant growth-promoting effect on bifid bacteria, especially, pullulan having a remarkable effect. Thus, the present applicants reached the present invention, which provides use of an agent which contains as an effective component pullulan together with or without dextran, for the manufacture of a medicament for the maintenance/improvement of health and for disease which can be treated and/or prevented by intestinal bifid bacteria in human or other animals, wherein the agent contains no hemicellulose when containing pullulan.

The field tests also revealed that the agent had the following features: (i) it preferentially promotes the growth of bifid bacteria in intestines to induce organic acid production, lower intestinal pH level, induce an appropriate peristalsis and also to relieve intestinal disorder; and (ii) it induces to an increment of feces and shows a much stronger intestinal disorder-relieving effect which had been deemed to be an activity of dietary fibers.

It was found that unlike oligosaccharides pullulan and dextran were ideal saccharides directed to promote the growth of bifid bacteria because they showed a much less individual difference when tested for growth-promoting effect on bifid bacteria. This means that they exert a satisfactorily high effect when taken in an small dose, while they have no fear of causing diarrhea when the dose increases to a relatively high level.

The growth-promoting agent according to the invention is that which contains pullulan and/or dextran and may promote the growth of bifid bacteria when administered orally or in the form of intubation feeding. Such growth-promoting agent can be prepared by a suitable method wherein pullulan and/or dextran is processed, if necessary, together with other one or more appropriate substances into solid, semisolid or liquid form.

Examples of such substances are nutrient substances such as amino acids, proteins, unsaturated fatty acids, lipids, saccharides, vitamins and minerals; edible extracts such as chlorella extract, yeast extract, mushroom extract, ginseng extract, aroe extract, snapping turtle extract, kelp extract, oyster extract, bonito extract and beef extract; edible fluids such as fruit juice, vegetable juice, soybean milk, milk, milk serum and yogurt; edible pastes such as sesami paste, nuts paste, bread dough, gelatinized starch paste and "nama-an (not sweetened bean jam)"; useful microorganisms such as bifid bacteria, lactic acid bacteria and yeasts; oligosaccharides capable of promoting the growth of bifid bacteria, such as lactulose, raffinose, lactosucrose, isomaltooligosaccharide, fructooligosaccharide, galactooligosaccharide and xylooligosaccharide; dietary fibers such as "fusuma (cereal bran)", pectin, locastbean gum, guar gum, mannan, agar, alginic acid, carragheenan, and Polydextrose®; and other substances such as medicine for stomach and bowels and those for relieving intestinal disorder or coprostasis. If necessary, one or more of these substances can be freely chosen and incorporated into final products to impart other one or more activities thereto, as well as to augment their growth-promoting effect.

Particularly, one or more oligosaccharides capable of promoting the growth of bifid bacteria can be advantageously incorporated in the range of about 0.1-10 folds of pullulan and/or dextran, on the dry solid basis.

In addition to the above, other one or more substances, for example, taste-imparting agent, coloring agent, flavor-imparting agent, stabilizer and filler can be favorably added to meet to consumers' taste preference during the preparation of the growth-promoting agent.

Both pullulan and dextran used in the invention are known water-soluble, low-viscous homoglucans having typically a molecular weight in the range of 2,000-5,000,000, preferably, in the range of about 10,000-2,000,000. Especially, pullulan has the feature that it is highly tolerant to salt, stable in salt solution, high in adhesion, binding ability, and superior in film-forming ability, moldability and water-solubility.

To incorporate pullulan and/or dextran, they are subjected in solid or solution form to one or more methods known per se, for example, mixing, kneading, dissolving, soaking, dispersing, applying, coating, spraying, injecting, solidifying and molding.

The growth-promoting agents thus obtained would find extensive uses dependently on their type, administration route, dose, content of pullulan and/or dextran and administration frequency. Generally, it is recommendable to administer about 0.1-100 g/day/adult, preferably, about 0.5-20 g/day/adult every day in terms of the content of pullulan and/or dextran in the agent.

Ingestion of the agent preferentially promotes the growth of bifid bacteria in intestines, more particularly, in large intestine to induce the production of organic acids such as acetic acid and lactic acid and this lowers the intestinal pH level. Furthermore, the agent suppresses the growth of infectious- and putrefactive-bacteria and this suppresses harmful substances which are easily produced during the metabolism of certain substances such as amino acids and proteins. Furthermore, besides adequately stimulating intestines to induce an appropriate peristalsis, the ingestion of the agent leads to an increment of feses and this remarkably augments its intestine disorder-relieving effect and prevents constipation.

Accordingly, the growth-promoting agent according to the invention can be favorably used in human to maintain and improve health and beauty, restore health during and after suffering from diseases and also to prevent and treat diseases such as geriatric diseases, hyperammonemia and hepatic encephalopathy, regardless of age and sex distribution.

Furthermore, the agent does exert its activities in domestic animals such as pig, dog and cat; fowls such as canary, parrakeet and chicken; and other animals such as honey bee, silkworm and fishes. Because of this, the agent can be favorably used in the prevention of infection and diarrhea, promotion of fattening, egg-laying, spawning and oviposition, and suppression of unpleasant smell from feces.

The following experiments will explain in detail the present invention.

### Experiment

### Effect of homoglucans on the promotion of bifid bacterium growth

Eight male volunteers (33.4 year old and 62.8 kg on average) received 10 g either of pullulan, dextran and soluble starch, predissolved in hot soup, at lunch time once every day over a period of 14 days.

In this experiment, after finishing an ingestion experiment on pullulan and a subsequent controlling period (14 days), an additional ingestion experiment on dextran and a further ingestion experiment on soluble starch were carried out similarly in this order. Before and after 14-day ingestion, the volunteers' feces were checked for their wet weight (g/day), relative change in wet weight, pH, total cell count/g of fresh feces, bifid bacterial ratio (%) against the total cell count, and relative change in bifid bacterial count, followed by calculating averages for the volunteers.

Among these items, the total cell count was determined by the method in Tomotari Mitsuoka, A color Atlas of Anaerobic Bacteria, pp.53-65, published by Kabushiki Kaisha Sobunsha, Tokyo, Japan (1984): The colonies obtained by using 13 different media excluding M10 medium were checked for their bacterial group (genus) and cell count. The highest cell count for each bacterial group among those attained in these media was adopted as the real cell count, while the sum of cell counts for each bacterial groups was adopted as the total cell count in feces. The bifid bacterial ratio (%) was expressed by the percentage (%) of the cell count for bifid bacteria (Bifidobacterium) against the total cell count.

The relative change in bifid bacterial count was expressed as the ratio of the total bifid bacterial count found after 14-day ingestion against that found before ingestion as 100.

The results were as shown in Tables, wherein Table 1 shows the changes in bacterial floras found in the volunteers' feces; and Table 2 shows the change in wet weight, pH and total bifid bacterial count in the volunteers' feces.

**Table 1**

| Changes of bacterial floras in volunteers' feces | | | | | | |
|---|---|---|---|---|---|---|
| Bacterial group | Pullulan | | Dextran | | Soluble starch | |
| | Before | After | Before | After | Before | After |
| Bifidobacterium | 9.9±0.4 | 10.3±0.3 | 9.9±0.4 | 10.2±0.4 | 9.9±0.3 | 9.7±0.2 |
| Bacteroidaceae | 10.6±0.2 | 10.6±0.1 | 10.7±0.3 | 10.7±0.2 | 10.6±0.2 | 10.6±0.2 |
| Eubacterium | 10.1±0.3 | 10.2±0.3 | 10.0±0.2 | 10.1±0.1 | 9.8±0.3 | 9.7±0.2 |
| Peptococcaceae | 9.6±0.5 | 9.3±0.3 | 9.7±0.4 | 9.8±0.3 | 9.7±0.2 | 9.7±0.5 |
| Others | 8.7±1.2 | 8.8±0.9 | 8.6±1.5 | 8.4±1.4 | 9.1±1.0 | 8.7±1.3 |
| Total cell count | 10.8±0.3 | 10.9±0.2 | 10.8±0.3 | 10.9±0.2 | 10.8±0.2 | 10.7±0.2 |
| Note: The values are cell counts per g feces, expressed by Log₁₀ mean value±SD. | | | | | | |

**Table 2**

| Changes in weight, pH and total bifid bacterial count of volunteers' feces | | | | | | |
|---|---|---|---|---|---|---|
| Items | Pullulan | | Dextran | | Soluble starch | |
| | Before | After | Before | After | Before | After |
| Wet weight of feces (g/day) | 129±30 | 188±35 | 127±28 | 144±30 | 130±33 | 129±38 |
| Relative change of wet weight of feces | 100 | 146 | 100 | 113 | 100 | 99 |
| pH | 6.7±0.4 | 5.9±0.3 | 6.6±0.5 | 6.1±0.4 | 6.7±0.4 | 6.6±0.4 |
| Total cell count per g of fresh feces | 10.8±0.3 | 10.9±0.2 | 10.8±0.3 | 10.9±0.2 | 10.8±0.2 | 10.7±0.2 |
| Bifid bacterial count per g of fresh feces | 9.9±0.4 | 10.3±0.3 | 9.9±0.4 | 10.2±0.4 | 9.9±0.3 | 9.7±0.2 |
| Percentage (%) of bifid bacteria against total cell count | 12.0 | 24.8 | 13.2 | 18.5 | 11.5 | 10.1 |
| Relative change of total bifid bacterial count | 100 | 358 | 100 | 215 | 100 | 82 |
| Judgement | Present invention | | Present invention | | Control | |
| (Note) The cell counts per g feces are expressed by Log₁₀ mean value±SD and other values are expressed by mean values or mean values±SD. | | | | | | |

As evident from the results in Tables 1 and 2, it was found that, unlike isoluble starch, pullulan and dextran did increase the amount of feces per day, bifid bacterial count/g feces, bifid bacterial ratio against total cell count by about 1.5-2 folds and total bifid bacterial count by about 2-4 folds when ingested, as well as decreasing the pH level in feces by about 0.5-1.0. Also was found that pullulan was superior to dextran with respect to any of these items.

These confirmed that pullulan and dextran has a function as dietary fiber, as well as exerting their inherent growth-promoting activity on bifid bacteria.

Accordingly, the growth-promoting agent for bifid bacteria containing pullulan and/or dextran as an effective component has both functions as growth-promoting agent for bifid bacteria and dietary fiber. This renders the agent very useful in the maintenance and improvement of health and beauty, prevention of geriatric diseases such as hypertension, diabetes, myocardial infarction and malignant tumors, restoration of health during and after suffering from diseases, and treatment and prevention of diseases including hyperammonemia and hepatic encephalopathy.

Several embodiments of growth-promoting agents for bifid bacteria directed to oral administration or intubation feeding will be explained hereinafter.

### Example 1

### Orally-ingestible growth-promoting agent for bifid bacteria

One hundred parts by weight of pulverized corn bran, 100 parts by weight of pullulan having a molecular weight of about 300,000, 5 parts by weight of "PANORUP®", an oligosaccharide syrup commercialized by Hayashibara Co., Ltd., Okayama, Japan, and an appropriate amount of water were mixed to homogeneity, and the mixture was fed to a granulator to obtain a granular growth-promoting agent for bifid bacteria.

Although the product can be orally ingested intact, one can process it with a tabletting machine into much more easily ingestible form, if necessary.

The product may be dissolved in "okayu (rice gruel)", milk, oatmeal, soup and juice, prior to its use. Furthermore, one can add the product to other materials in the cooking and processing of various orally-ingestible products such as health foods, pharmaceuticals, feeds and pet foods.

Since the product exerts both growth-promoting effect on bifid bacteria and function as a dietary fiber, it is favorably usable in the maintenance and promotion of health and beauty, prevention of geriatric diseases, restoration of health during and after suffering from diseases, and treatment and prevention of certain diseases including hyperammonemia and hepatic encephalopathy.

The product would find additional uses in the prevention of infection and diarrhea, and promotion of fattening in domestic animals including poultry, as well as in the suppression of unpleasant smell from their feces.

### Example 2

### Orally-ingestible growth-promoting agent for bifid bacteria

Fifteen parts by weight of pullulan having a molecular weight of about 200,000, 100 parts by weight of "SUNMALT®", a pulverized maltose commercialized by Hayashibara Co., Ltd., Okayama, Japan, 40 parts by weight of powdered sugar, 20 parts by weight of shortening, 1.5 parts by weight of sugar ester, 6 parts by weight of coffee powder, 3 parts by weight of milk powder, 4 parts by weight of gelatin preswollen in 3 volumes of hot water, 70 parts by weight of isomaltooligosaccharide syrup, and 15 parts by weight of water were mixed, fed to a kneader, molded and packed to obtain a soft candy-type product having a coffee taste.

The product, which meets to users' taste preference and has both growth-promoting effect on bifid bacteria and function as a dietary fiber, is orally ingestible and favorably usable in the maintenance and promotion of health and beauty, as well as in or for growth-promoting agent for bifid bacteria.

### Example 3

### Orally-ingestible growth-promoting agent for bifid bacteria

Two hundred and twenty parts by weight of sucrose, 220 parts by weight of "COUPLING SUGAR®", a sucrose bound starch syrup commercialized by Hayashibara Co., Ltd., Okayama, Japan, 60 parts by weight of dextran having a molecular weight of about 500,000, and an appropriate amount of food dye were placed in a vessel having a flat bottom, added with 210 parts by weight of water, boiled down while mixing, and concentrated till the temperature of the concentrate reached 117°C. The condensate was then taken out from the vessel, added with 30 parts by weight of powdered sucrose and 0.1 part by weight of menthol, cooled, molded and packed to obtain an edible chewing gum type-product having a mint taste.

Similarly as the product in Example 3, the product meets to users' taste preference and has both growth-promoting effect on bifid bacteria and function as a dietary fiber. Thus, the product is favorably usable in the maintenance and promotion of health and beauty.

### Example 4

### Orally-ingestible growth-promoting agent for bifid bacteria

One thousand parts by weight of water was heated up to 70°C, and added with 100 parts by weight of HMP (rapid-set pectin) and 1,000 parts by weight of sucrose bound starch syrup. When the mixture began to boil, it was further added with 8,900 parts by weight of sucrose bound starch syrup and 100 parts by weight of pullulan having a molecular weight of about 10,000, followed by heating till the temperature of the mixture reached 104°C. The resultant was then cooled to 95°C, added with appropriate amounts of lemon flavor and food dye, admixed with a citric acid solution which had been prewarmed to 60°C to give pH 3.3, distributed, cooled and packed to obtain a lemon jelly type-product

The agent having both growth-promoting effect on bifid bacteria and function as dietary fiber is favorably usable in the maintenance and promotion of health and beauty.

### Example 5

### Orally-ingestible growth-promoting agent for bifid bacteria

Ten thousand parts by weight of defatted milk was pasteurized at 80°C for 20 minutes, cooled to 40°C, added with 300 parts by weight of lactic acid bacteria as starter, and fermented at 35-37°C for 10 hours. The resultant was then homogenized, mixed with 4,000 parts by weight of isomerized syrup, 2,000 parts by weight of sucrose and 170 parts by weight of pullulan having a molecular weight of about 20,000, and pasteurized by keeping the temperature at 70°C.

After cooling, the resultant mixture was added with a small amount of flavor, and bottled to obtain a lactic acid beverage type product.

The product, which exerts both growth-promoting effect on bifid bacteria and function as a dietary fiber, is effective in the reduction of intestinal pH level, suppression of putrefactive bacteria, and prevention of constipation.

### Example 6

### Orally-ingestible growth-promoting agent for bifid bacteria

Ten parts by weight of pullulan having a molecular weight of about 100,000 was dissolved in 1,000 parts by weight of a vegetable juice mainly from tomato, and the mixture was pasteurized by heating and canned in conventional manner to obtain a juice type product.

The product is an ideal health beverage because it supplements vitamins and minerals originated from the vegetable juice and exerts both growth-promoting effect on bifid bacteria and function as a dietary fiber.

### Example 7

### Orally-ingestible growth-promoting agent for bifid bacteria

Twenty-five parts by weight of pullulan having a molecular weight of about 80,000, 25 parts by weight of lactosucrose, 1 part by weight of calcium tertiary phosphate, 1 part by weight of sugar ester and powdered flavor were mixed to homogeneity, after which the resultant mixture was fed to a tabletting machine in conventional manner to obtain a product in tablet form, about 350mg each.

The product exerts both growth-promoting effect on bifid bacteria and function as a dietary fiber when ingested in an amount of about 1-40 tablets/day/adult, preferably, about 2-20 tablets/day/adult.

### Example 8

### Orally-ingestible growth-promoting agent for bifid bacteria

Thirty parts by weight of pullulan having a molecular weight of about 100,000, 18 parts by weight of MABIT®, an anhydrous crystalline maltitol commercialized by Hayashibara Shoji, Inc, Okayama, Japan, 2 parts by weight of maltose powder containing 0.1 w/w % of bifid bacteria, 1 part by weight of calcium tertiary phosphate, 1 part by weight of sugar ester and an appropriate amount of powdered flavor were mixed to homogeneity. The resultant mixture was then fed to a tabletting machine in usual manner to obtain a product in tablet form, about 400mg each.

The product exerts both growth-promoting effect on bifid bacteria and function as a dietary fiber when ingested in an amount of about 1-40 tablets/day/adult, preferably, about 2-20 tablets/day/adult.

### Example 9

### Orally-ingestible growth-promoting agent for bifid bacteria

Twenty parts by weight of dextran having a molecular weight of about 90,000, 30 parts by weight of lactosucrose, 1 part by weight of calcium tertiary phosphate, 1 part by weight of sugar ester and appropriate amounts of powdered food dye and powdered flavor were mixed to homogeneity. The resultant mixture was then fed to a tabletting machine in usual manner to obtain a product in tablet form, about 680mg each.

The product exerts both growth-promoting effect on bifid bacteria and function as a dietary fiber when ingested in an amount of about 1-40 tablets/day/adult, preferably, about 2-20 tablets/day/adult.

### Example 10

### Growth-promoting agent for bifid bacteria directed to intubation feeding

Twenty-five g aliquots of a composition containing 580 parts by weight of "FINETOSE®", a crystalline alpha-maltose commercialized by Hayashibara Co., Ltd., Okayama, Japan, 190 parts by weight of dehydrated egg, 209 parts by weight of defatted milk powder, 15 parts by weight of pullulan having a molecular weight of about 50,000, 4.4 parts by weight of sodium chloride, 1.85 parts by weight of potassium chloride, 4 parts by weight of magnesium sulfate, 0.01 part by weight of thiamine, 0.1 part by weight of sodium ascorbate, 0.6 parts by weight of vitamin E acetate and 0.04 parts by weight of nicotinamide were distributed to small laminated aluminum bags which were then heat-sealed to obtain a ready-mix type product.

The product necessarily does not require cold storage and is stable over a long period even at ambient temperature and superior in solubility and dispersibility.

In use, one bag of the product is dissolved in about 150-300 ml of hot water, and the resultant solution is ingested to patients by intubation feeding through their nasal cavity, gullet or stomach. Such ingestion exerts both growth-promoting effect on bifid bacteria and function as a dietary-fiber and these help patients to restore and promote their health.

More particularly, the product promotes the growth of bifid bacteria in the large intestine, lowers its pH level and suppresses the formation of harmful substances which originate from decomposing substances.

Furthermore, the ingestion of the product leads to an increment of feces and this prevents constipation from which patients often suffer.

In addition to use in human, the product is favorably usable in domestic animals as a growth-promoting agent directed to oral ingestion and intubation feeding.

### Example 11

### Growth-promoting agent for bifid bacteria directed to intubation feeding

Four hundred g aliquots of a composition containing 16.5 parts by weight of crystalline alpha-maltose, 4.05 parts by weight of sugar, 3.2 parts by weight of large mandarin orange juice powder, 1.0 part by weight of dextran having a molecular weight of about 40,000, 0.11 parts by weight of citric acid, 0.02 parts by weight of ascorbic acid, and 0.1 part by weight of powdered orange flavor were distributed to cans with screw caps which were then sealed to obtain a ready-mix type product.

Similarly as the product in Example 10, the product is excellent in stability and solubility.

In use, about 25 g of the product is dissolved in about 100-150 ml of hot water, and the resultant solution is ingested to patients by intubation feeding similarly as in Example 10. Such ingestion exerts both growth-promoting effect on bifid bacteria and function as a dietary fiber and these help patients to restore their health.

As described in the above, the present inventors found that pullulan and dextran exerted a growth-promoting effect on bifid bacteria in vivo. The present invention is to provide a growth-promoting agent for bifid bacteria containing pullulan and/or dextran as an effective component.

Oral ingestion and intubation feeding of the agent preferentially promote the growth of bifid bacteria in intestines to induce the production of organic acids such as acetic acid and lactic acid, and this lowers intestinal pH level. Such ingestion and intubation feeding also suppress the growth of infectious- and putrefactive-bacteria, as well as suppressing the formation of harmful substances which may occur during the metabolism of amino acids and proteins.

Furthermore, the agent appropriately stimulates the intestines to restore its inherent peristalsis and leads to an increment of feces. These are extremely effective in relieving intestinal disorder, as well as in preventing constipation.

Accordingly, the agent is favorably usable in the maintenance and promotion of health and beauty, prevention of geriatric diseases such as hypertension, diabetes, myocardial infarction and malignant tumors, restoration of health during and after suffering from diseases, and treatment and prevention of certain diseases including hyperammonemia, hepatic encephalopathy.

In addition, the agent also promotes the growth of bifid bacteria in domestic animals including poultry and this renders the agent favorably usable in the prevention of infection and diarrhea and promotion of fattening, egg-laying, spawning and oviposition, as well as in reduction of unpleasant smell from their feces.

Since pullulan and dextran are non-toxic, harmless, high in safeness and producible in an industrial-scale, the growth-promoting agent for bifid bacteria according to the invention would have a great significance in pharmaceutical, health food, and feed industries.

## Claims

1. Use of an agent which contains as an effective component pullulan together with or without dextran, for the manufacture of a medicament for the maintenance/improvement of health and for disease which can be treated and/or prevented by intestinal bifid bacteria in human or other animals, wherein the agent contains no hemicellulose when containing pullulan.

2. Use as claimed in claim 1, wherein the molecular weight of said pullulan is in the range of 2,000-5,000,000.

3. Use as claimed in claim 1, wherein said dextran is present in the agent and has a molecular weight in the range of 2,000-5,000,000.

4. Use as claimed in any one of claims 1 to 3, wherein the dose of the agent is in the range of about 0.1-100 g/day/adult with respect to the amount of pullulan and/or dextran in said agent.

5. Use as claimed in any one of the preceding claims wherein said agent additionally contains a member selected from the group consisting of nutrient agent, vitamin, mineral, edible extract, edible fluid, edible paste, microorganism, oligosaccharide, dietary fiber, pharmaceutical, and mixtures thereof.

6. Use as claimed in claim 5, wherein the weight ratio of said oligosaccharide to pullulan and/or dextran is in the range of about 0.1-10, on a dry solid basis.

7. Use as claimed in claim 5 or claim 6, wherein said oligosaccharide is a member selected from the group consisting of laculose, raffinose, lactosucrose (or 0-β-D-galactosyl-(1→4)-O-α-D-glucosyl-(1←2)-β-D-fructoside), isomaltooligosaccharide, fructooligosaccharide, galactooligosaccharide and xylooligosaccharide.

8. Use as claimed in any one of the preceding claims, wherein the agent is in the form of solid, semisolid or liquid.

9. Use as claimed in any one of the preceding claims, wherein the agent is used for oral administration or intubation feeding.

10. Use as claimed in any one of the preceding claims, wherein said bifid bacteria are of the genus *Bifidobacterium*.

11. Use as claimed in any one of claims 1 to 10 in prevention of geriatric diseases.

12. Use as claimed in any one of claims 1 to 10 in health restoration during or after disease.

13. Use as claimed in any one of claims 1 to 10 in the prevention and/or treatment of hyperammonemia and hepatic encephalopathy.

14. Use as claimed in any one of claims 1 to 10 in treating animals.

## Patentansprüche

1. Verwendung eines Mittels, das als Wirkstoffkomponente Pullulan zusammen mit oder ohne Dextran enthält, zur Herstellung eines Medikaments für die Erhaltung/Verbesserung der Gesundheit und für eine Erkrankung, die durch Darm-Bifidusbakterien beim Menschen oder anderen Tieren behandelt und/oder verhindert werden kann, wobei das Mittel keine Hemicellulose enthält, wenn es Pullulan enthält.

2. Verwendung nach Anspruch 1, worin das Molekulargewicht des Pullulans im Bereich von 2.000 - 5.000.000 liegt.

3. Verwendung nach Anspruch 1, worin das Dextran im Mittel vorhanden ist und ein Molekulargewicht im Bereich von 2.000 - 5.000.000 aufweist.

4. Verwendung nach einem der Ansprüche 1 bis 3, worin die Dosis des Mittels im Bereich von etwa 0,1 - 100 g/Tag/Erwachsener im Hinblick auf die Menge Pullulan und/oder Dextran im Mittel liegt.

5. Verwendung nach einem der vorangegangenen Ansprüche, worin das Mittel zusätzlich einen Bestandteil enthält, der aus der Gruppe Nährmittel, Vitamin, Mineral, eßbarer Extrakt, eßbare Flüssigkeit, eßbare Paste, Mikroorganismus, Oligosaccharid, Diätfaser, Pharmazeutikum und Mischungen daraus ausgewählt ist.

6. Verwendung nach Anspruch 5, worin das Gewichtsverhältnis des Oligosaccharids zum Pullulan und/oder Dextran im Bereich von etwa 0,1 - 10, auf trockener Feststoffbasis, liegt.

7. Verwendung nach Anspruch 5 oder 6, worin das Oligosaccharid ein Bestandteil ist, der aus der Gruppe Laculose, Raffinose, Lactosucrose (oder 0-β-D-Galactosyl-(1→4) - O-α-D-glucosyl-(1←2)-β-D-fructosid), Isomaltooligosaccharid, Fructooligosaccharid, Galactooligosaccharid und Xylooligosaccharid ausgewählt ist.

8. Verwendung nach einem der vorangegangenen Ansprüche, worin das Mittel in Form eines Feststoffs, eines Halbfeststoffes oder einer Flüssigkeit vorliegt.

9. Verwendung nach einem der vorangegangenen Ansprüche, worin das Mittel für die orale Verabreichung verwendet wird oder über Intubation zugeführt wird.

10. Verwendung nach einem der vorangegangenen Ansprüche, worin die Bifidusbakterien von der Gattung Bifidobacterium stammen.

11. Verwendung nach einem der Ansprüche 1 bis 10, zur Vorbeugung von geriatrischen Erkrankungen.

12. Verwendung nach einem der Ansprüche 1 bis 10, zur Wiederherstellung der Gesundheit während oder nach Erkrankungen.

13. Verwendung nach einem der Ansprüche 1 bis 10, zur Vorbeugung und/oder Behandlung von Hyperammonemie und Leber-Enzephalopathie.

14. Verwendung nach einem der Ansprüche 1 bis 10, zur Behandlung von Tieren.

## Revendications

1. Utilisation d'un agent qui contient du pullulanne, comme composant actif, conjointement avec du dextrane ou sans dextrane, pour la fabrication d'un médicament pour le maintien/l'amélioration de la santé et contre une maladie qu'on peut traiter et/ou prévenir au moyen de bifidobactéries intestinales chez l'homme ou d'autres animaux, dans laquelle ledit agent ne contient pas d'hémicellulose quand il contient du pullulane.

2. Utilisation selon la revendication 1, dans laquelle le poids moléculaire dudit pullulanne est compris entre 2 000 et 5 000 000.

3. Utilisation selon la revendication 1, dans laquelle ledit dextrane est présent dans l'agent et a un poids moléculaire compris entre 2 000 et 5 000 000.

4. Utilisation selon l'une quelconque des revendications 1 à 3, dans laquelle la dose d'agent est comprise entre environ 0,1 et 100 g/jour/adulte par rapport à la quantité de pullulanne et/ou de dextrane dans ledit agent.

5. Utilisation selon l'une quelconque des revendications précédentes, dans laquelle ledit agent contient, en outre, un élément choisi dans l'ensemble constitué par un agent nutritif, une vitamine, un minéral, un extrait comestible, un fluide comestible, une pâte comestible, un micro-organisme, un oligosaccharide, une fibre diététique, une substance pharmaceutique, et des mélanges de ceux-ci.

6. Utilisation selon la revendication 5, dans laquelle le rapport en poids dudit oligosaccharide au pullulane et/ou au dextrane est compris entre environ 0,1 et 10, sur la base du solide sec.

7. Utilisation selon la revendication 5 ou la revendication 6, dans laquelle ledit oligosaccharide est un élément choisi dans l'ensemble constitué par le lactulose, le raffinose, le lactosaccharose (ou O-β-D-galactosyl-(1―>4)-O-α-D-glucosyl-(1<―2)-β-D-fructoside), un isomalto-oligosaccharide, un fructo-oligosaccharide, un galacto-oligosaccharide et un xylo-oligosaccharide.

8. Utilisation selon l'une quelconque des revendications précédentes, dans laquelle l'agent est à l'état solide, semi-solide ou liquide.

9. Utilisation selon l'une quelconque des revendications précédentes, dans laquelle on utilise l'agent pour une administration orale ou pour une alimentation par intubation.

10. Utilisation selon l'une quelconque des revendications précédentes, dans laquelle lesdites bifidobactéries sont du genre *Bifidobachecium*.

11. Utilisation selon l'une quelconque des revendications 1 à 10 pour la prévention des maladies gériatriques.

12. Utilisation selon l'une quelconque des revendications 1 à 10 pour rétablir la santé pendant ou après une maladie.

13. Utilisation selon l'une quelconque des revendications 1 à 10 pour la prévention et/ou le traitement de l'hyperammoniémie et de l'encéphalopathie hépatique.

14. Utilisation selon l'une quelconque des revendications 1 à 10 pour traiter les animaux.
